# EUROPEAN PATENT SPECIFICATION

(11) **EP 1 682 008 B1**
(45) Date of publication and mention of the grant of the patent: **30.05.2007**
(21) Application number: 04797494.4
(22) Date of filing: 05.11.2004
(51) Int. Cl.: A61B 17/00

(54) **A FIXATOR FOR REPAIRING OF LONG BONE FRACTURES**
FIXATOR ZUR REPARATUR VON RÖHRENKNOCHENFRAKTUREN
FIXATEUR POUR REPARER DES FRACTURES DES OS LONGS

(30) Priority: 05.11.2003 EE 200300492
(43) Date of publication of application: 26.07.2006
(73) Proprietor: Tartu Ülikool, EE50090 Tartu (EE)
(72) Inventor: ANDRIANOV, Vladimir, EE51004 Tartu (EE); LENZNER, Aleks, EE51003 Tartu (EE); HAVIKO, Tiit, EE50407 Tartu (EE)
(74) Representative: Sarap, Margus
(86) International application number: PCT/EE2004/000007
(87) International publication number: WO 2005/044121

(56) References cited:
- US-A- 3 680 553
- US-A- 4 054 955
- US-A- 5 941 878
- US-A1- 2002 143 337
- US-B1- 6 379 359

## Description

### TECHNICAL FIELD

The present invention relates to the field of orthopaedic and veterinary surgery. Particularly it describes the devices for extramedullary and intramedullary fixing of the fracture of the long tubular bones.

### BACKGROUND ART

The approach to the pathophysiology of bone fracture has significantly changed in the last decades. The knowledge of fracture healing and management have improved in veterinary as in traumatology. In animals, it is of utmost importance for the damaged bone to recover as fast as possible, since it is impossible to keep the animal immobilized through the whole healing period. The quicker and more intensive the regeneration of the bone tissue is, the better is the functional recovery of the limb and the fewer are the complications. The most important components in fracture treatment are accurate anatomical repositioning of the bone fragments, stabile fixation and early mobilization of the limb. Implementation of osteosynthesis in practice requires special knowledge, experience and technical skills, and specific materials and instruments. Different types and locations of fractures require different surgical techniques and specific fixing devices. In the present field there exists a demand for devices and methods that simultaneously meet the following requirements:
- the bone fragments must be strongly fixed;
- the application of the fixing elements has to be minimally traumatic and the surgery must be performed with as minimal soft tissue damage as possible;
- the fixing construction must be compact, strong and sustain to the limit the structure and the vital functions of the bone tissue;
- the fixation must not inhibit nor block the regeneration of bone-tissue i.e. formation of bone callus, it must not irritate the surrounding tissues;
- the fixing element has to enable the animal to use the damaged limb right after the surgery for reduction of the possibility of complications;
- the fixator must be technically simply realizable, to consist of universal details, so it can easily be disassembled and reused;
- the design of the fixator must be such that it can be made in small, medium and large sizes;
- the use of the fixator must be simple and quick, without extensive time and complicated equipment;
- before and after osteosynthesis, the fixator must evenly share the load between itself and the bone fragments.

In the examples, the present invention is used in orthopaedic surgery of small animals, however, the above-mentioned principles apply to all mammals, including big domestic animals and man. For a person skilled in the art, the described methods and devices, with small adjustments, are easily applicable on bigger animals, such as larger breeds of dogs, and human.

In modern orthopaedic surgery of small animals, in addition to conservative treatment of fractures of tubular bones, various surgical methods are being used, including intramedullary and extramedullary fixation.

This is conditioned by the varying nature of bone fractures, whereby any selected method cannot be maximally effective for all kinds of tubular bone fractures.

As examples of intramedullary fixation the patent applications US2003069581A1 "Universal intramedullary nails, systems and methods of use thereof' and US20020183753A1 "Rod implant for osteosynthesis of long bones" may be drawn. With such treatment, a typical procedure will be insertion or implantation of a nail or rod into the intramedullary channel of the broken bone, such as the nail or rod extends both sides of the point of fracture. After that screws are screwed through holes in the nail (rod) such as to fasten into the bone on both sides of the nail (rod). Thereby the bone or bone fragments are fixated and rotation and lateral movement prevented.

This procedure has its drawbacks, such as destruction of the vascular system caused by milling and boring of the bone channel. Retarded healing and contingent complications have been demonstrated in clinical settings.

The extramedullary fixation by bone plates is widespread in veterinary surgery and traumatology. They were taken into usage in the 1960s. There are some drawbacks to this method as well such as excessive rigidity contributing to osteoporosis. Also, there are very many steps in this method, which makes it difficult in lack of time. The fixation of a multitude of screws is also an additional trauma for the vascular and nervous system. In addition or as a replacement of the screws, an intramedullary nail can also be fixed with C-clamps-on plate.

For the first time a combined fixator was used by A.Seppo in 1979, in man (1). The purpose was to mechanise the accurate positioning of bone fragments without scraping and injuring the location and surroundings of the fracture. It also eliminated the need for a plaster splint. The latter impairs the correct mineralization of tissue in long-term usage. It further sustained the kinaesthetic and support function of the uninjured part of bone immediately after surgery. The device takes advantage of bow-shaped rods, which are in tandem and rest upon bone cortex from inside with their ends. There is a granted patent US3680553 "Fixing apparatus for osteosynthesis of fractures of long tubular bones" for the described device. This is the most similar background art to the present invention and discloses the following features: a fixator for combined metal osteosynthesis for fracture repair of long tubular bones, wherein the fixator comprises periostal connecting coupling; the coupling has oblique planished holes, at least one curved rod and at least one cortical screw, whereby the diameter of the curved rod is chosen such to fit the curved rod through the oblique hole of the connecting coupling; the curved part of the curved rod is applied intramedullary, the other end or tail-end of the curved rod is applied onto the connecting coupling; the tail-end of the curved rod contains a hole for cortical screws and has a similar diameter to the hole for the cortical screw in the connecting coupling.

We would like to make a remark here that, as stated above, postoperative treatment in human and animals are based on completely different principles.

The inventors of the present invention have further elaborated the method of combined periostal and intramedullary osteosynthesis. Compared with Seppo's fixator, the device has been made simpler and easier to use. Preliminary tests show that it will suffice to use only one bow-shaped rod instead of a tandem. Also, fixing the rods to the supporting plate is substantially easier supporting.

### DISCLOSURE OF THE INVENTION

The present invention relates to a novel fixator which is used in a technique for fixation of fractures of long tubular bones in small animals. For proving the concept, a special fixator has been constructed that simultaneously contains elements of both intramedullary and extramedullary osteosynthesis.

The fixator consists of a periostal supporting plate, two curved rods and two cortical bone screws.

The curved rods are inserted into the intramedullary cavity such that the outside tails of curved rods will be fixed onto the supporting plate. In addition to the screw-plate interface a "forceps effect" between the curved rods and the plate fixates the bone (fragments). Thereby the wound is shorter than the distance between the ends of curved rods. The technical features and technique of using have been validated in cadavers of dogs and cats, and tested in experimental animals (rabbits). A person skilled in the art can adapt the invention easily for bigger animals and man. The parts of the fixator can be made in different sizes and forms, according to the different bones.

The invention adds to the existing field of art a simple device, which is easy to make and apply. For patients, it offers a smaller surgical trauma, faster recovery and better conveniences.

The objects of the invention are achieved by a fixation according to claim 1. Preferred embodiments are set forth in the dependent claims.

### BRIEF DESCRIPTION OF THE DRAWINGS

The present invention is described with reference to the drawings as follows, where:
Fig 1 shows the fixator, which simultaneously combines the elements of intramedullary and extramedullary osteosynthesis (the photo depicts a cat's thighbone, a part of which is opened to exhibit the intramedullary part);
Fig 2 shows the construction of the fixator from Fig 1;
Fig 2a shows the supporting plate and curved rods according to the invention;
Fig 3 shows the insertion of a curved rod through the supporting plate into the intramedullary channel;
Fig 4 to Fig 8 show the radiographs made after surgery in one patient (rabbit), bi-weekly;
Fig 9 to Fig 13 show the radiographs made after surgery in another patient (rabbit), bi-weekly;
Fig 14 shows the construction of a bridged combined fixator;
Fig 14a shows the supporting plate and curved rod according to the construction of a bridged combined fixator;
Fig 15 shows by radiographs the use of a bridged combined fixator in the treatment of dog's shinbone fracture, where:
   a), b) position of the fracture before and after the application of the fixator;
   c) after 1 month;
   d) after 2 months;
   e), f) after 3 months.

### DETAILED DESCRIPTION OF THE INVENTION

The purpose with the examples of the present invention was to investigate the effectivity of the developed procedure and devices of osteosynthesis in diaphyseal fractures, and to evaluate the recovery of function of the traumatic limb and the intensity of regeneration of the bone tissue in experimental animals. Example 1 describes the construction of the osteosynthesis device; example 2 describes the application surgery technique of the device; example 3 describes the tests after the surgery. Example 4 describes an improved, bridged device of osteosynthesis.

The fixator has proven effective in use with experimental animals. Even when one of the screws was unfastened, the curved rod shifted relatively little in the intramedullary channel the whole construction retaining its fixing and stabilising function. Herewith one must consider the fragility of a rabbit's bones.

The device of the invention simultaneously fulfils the requirements for the treatment of tubular bone fractures:
- the bone fragments are strongly fixed, the load being distributed between the device and the bone fragments;
- the mounting of the fixator is less traumatic and sparing of the soft tissues;
- the fixator is compact, strong and sustains the structure and vital functions of the bone tissue;
- the fixation does not suppress nor stop the regeneration of the bone tissue i.e. the formation of bone callus, and does not irritate the surrounding tissues;
- the fixing construction allows the animal to use its limb immediately post-surgery, for reduction of the possibility of complications. The fixator is technically simply realisable and modified, the details can easily be disassembled, swapped and re-used.

An additional advantage is the minimum training required. In summary, it means faster and more complete recovery and better convenience to the patient. These features render an advantage to it compared with traditional osteosynthesis.

The inventors have experimented with a modification of the present invention, where two curved rods get fixed with only one screw to the supporting plate. In this case a dent has to be cut in the tail-end of each curved rod (which will lie in the centre of the supporting plate), where the screw will sit.

Such a device has been disclosed in a conference report. However, the inventors do not suggest to use such a solution, since animal experiments proved it mechanically unstable.

### Example 1. Design of the combined fixator.

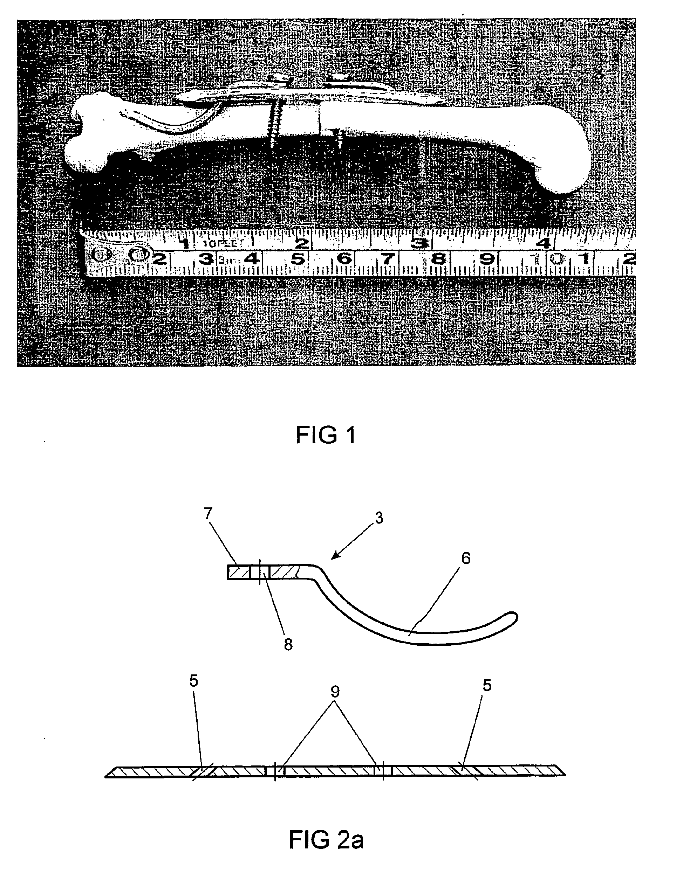

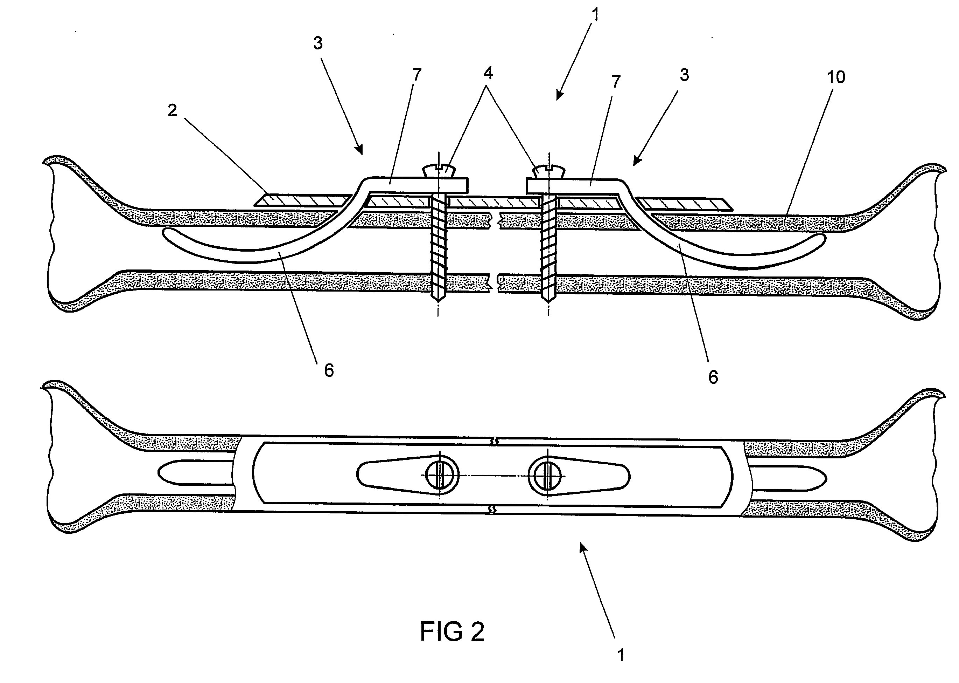

The fixator 1 is made of stainless steel and comprises a supporting plate 2, two curved rods 3, and two cortical bone screws 4 (drawing fig 2). The supporting plate 2 has a thickness of 2 mm and is convex in cross-section profile. The length of the supporting plate is approximately 2/5 of the length of the thighbone and the width is approximately 2/3 of the bone diameter. Within a distance of 10 mm from the ends of the supporting plate, two inclined holes at an angle of 45 degrees are drilled, with a diameter of 3.5 mm and pointing outside of the centre. The diameter of the inclined hole 5 should be in correspondence with the diameter of the curved rod curved rod 3. In trade literature, the curved rods may be called "curved rode", "anchors" or simply "rods". The bow-shaped part 6 of the curved rod 3 that gets guided into the intramedullary channel has a length of 25-27 mm, a width of 3-4 mm and a thickness of 2 mm, has an oval shape in cross-section and with the minimum taper of (0.5-0.8 mm) in the direction of periphery. Such a construction facilitates the removal of the curved rods 3 after the recovery of the fracture. The other end 7 (the tail-end) of the curved rod 3 having a measurement of 15x6x1.5 mm, is placed on the supporting plate 2 and has a flat shape and a similar convexity as the supporting plate 2. In the tail-end 7 of the curved rod 3 there is a hole 8 with a diameter of 3 mm that is necessary for the bone screws 4. In the supporting plate 2 there are also the holes 9 with a diameter of 3 mm, the said openings are being located within 8 mm from the centre of the supporting plate 2. The said openings are necessary for a simultaneous fixing of the curved rods 3 and the supporting plate 2 to the bone 10 by bone screws 4. Bone screws 4 of the cortical type are being used with the length of 18 mm and the diameter of 2.8 mm and they must extend through the both cortexes of the bone 10. The measurements for the above-mentioned fixator 1 are calculated for a rabbit of average size (weight 4-5 kg). If an animal of a different size is used, the measurements of the fixator 1 should be adjusted proportionally. It is done on the basis of X-ray radiography. The interval between the holes 5 milled and drilled into the supporting plate 2 are fixed and correspond to the measurements of the tail-end 7 of the curved rod 3. This enables to swap the curved rods 3 or replace them with different curved rods 3. Working with the fixator 1 is considerably easier and more comfortable if the surgeon has several supporting plates 2 and curved rods 3 with various measures.

The present fixator is provided for use on long bone fractures in the middle third of the diaphysis. The use is limited in the case of very fragmented fractures and in the case of extensive longitudinal splits of the bones.

### Example 2. The surgical technique of applying the combined fixator.

In addition to the ordinary kit for soft tissue surgery, the following instruments are used for performing the surgery: plate benders 2 pieces, bone forceps 2 pieces, bone drills with diameters of 2.0 mm and 3.5 mm, a screwing die 2.5 mm for cortical screws, two cortical screws with the diameter of 2.7 mm and with the length of 18 mm, a screwdriver, a light hammer and a drill.

### The surgical technique

The technique of mounting the fixator 1 is rather easy. A supporting plate 2 with appropriate measurements and curved rods 3 were made before surgery on the basis of X-ray radiography. It was considered that the profile of the supporting plate 2 would be in accordance with the profile of the test animal thighbone 10. The best way to determine it is by using bone samples. The length and radius of the curved rods 3 were selected on the basis of X-ray radiography considering the diameter of the intramedullary channel, to extend as far as possible beyond the fracture. The bending is correct if the curved rod 3 leans on the bone cortex in the three positions; in the tip of the curved rod 3, in the centre point of the curve 6 and in the region of the hole 5 milled into the supporting plate 2.

As anaesthetics (in rabbit), a combination of Domitor 400 µg/kg + Ketamin 20 mg/kg together in one syringe was used subcutaneously. The anaesthesia lasted between one and one and a half hour (2). A cannula was inserted into the ear vein, dripping during the operation 0.9% NaCl at one drop per second.

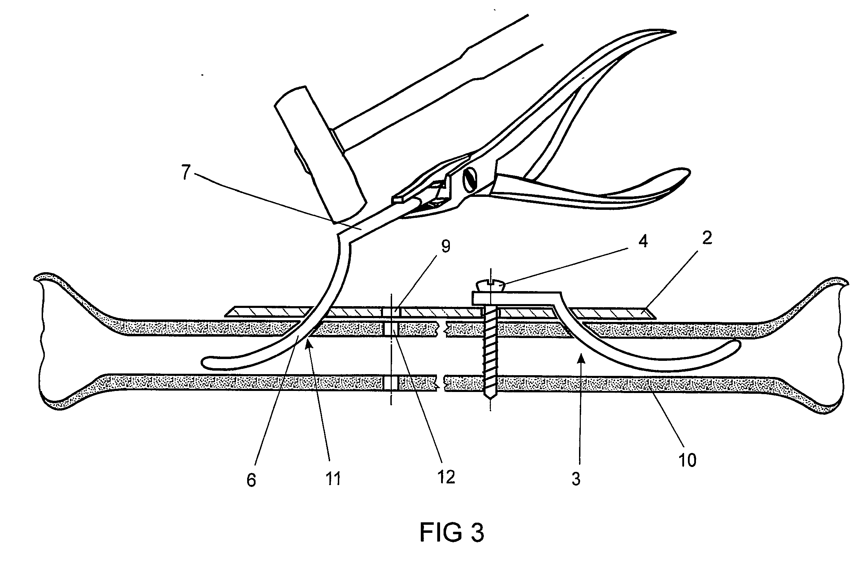

A lateral approach to the thighbone was used, where the craniolateral side of the diaphysis was surfaced from the soft tissues. The fixator was applied on the thighbone, maximally preserving the surrounding soft tissues (3). The diaphysis of the thighbone was disrupted with a thin pad saw. The ends of the fragments were reposited and the supporting plate 2 was mounted on the bone 10 while fixating with bone forceps. The bending of the supporting plate 2 was adjusted with plate benders if necessary during the surgery. The bone forceps must be placed on the bone 10 and the supporting plate 2 just before the milled hole 5. Such a placement of the bone forceps will not disturb subsequent manipulations. Further, in one of the fragments channel 11 was drilled at an angle of 45 degrees relative to the longitudinal axis of the bone, with a diameter of 3.5 mm, into the bone cortex. Drilling through the supporting plate 3, the hole 5 performs as a guide. Such a technique is very simple and provides the right angle and position for the channel 11. Further, the tail-end of the curved rod 3 is captured with a needle holder and with light hammer knocks the curved rod 3 is guided trough the supporting plate 2 and the channel 11, drilled into the cortex, so deep that the tail-end 7 of the curved rod 3 reaches the supporting plate 2 (drawing fig 3).

The bending of the curved rod 3 may be adjusted with plate benders in case of necessity such that the curved part 6 will move through the drilled channel 11 relatively freely (a medium pressure with a finger is sufficient) and a small tension originates in the curved rod 3 in the intramedullary channel. The holes 8 and 9 positioned in the tail-end 7 of the curved rod 3 and on the supporting plate 2 respectively must be on top of each other. Through the said holes, hole 12 with a diameter of 2 mm and spanning both cortexes is drilled. Further, the diameter of the bone 10 together with the supporting plate 2 and tail-end 7 of the curved rod 3 is measured, to determine the necessary size of the bone screw 4. The channel 11 is cut with a screwing die and the whole construction is fixed tightly with the bone screw 4 to the bone 10 (4). In the final stage of the screwing of the bone screw 4, the curved rod 3 that is situated in the intramedullary channel, will press against the supporting plate 2. Between the supporting plate 2 and the curved rod 3 a "forceps effect" is created, resulting in the fixator 1 being fixed to the bone 10. Such fixing will guarantee maximal stability to the construction, prevent rotation and forming of fractures. Similarly, the other curved rod 3 will be guided into the other fragment of the bone. The closure of the wound happens as usual. After the surgery, a control X-ray radiography was made in the mediolateral projection to evaluate the repositioning of the fragments and the position of the fixator 1. Carprophene (Rimadyl, Pfizer), 2.2 mg/kg was used orally as an analgetic. Immobilising constructions or cut bandage was not used. The operated animals were taken to a vivarium where they started leaning on the traumatised limb once the anaesthesic effect passed. In the described test series nine rabbits were operated using the same technique.

### Example 3. Post surgery control.

During the first post-surgery week a clinical evaluation of the limb was made every day. The setons were removed from the wound after 10 days. A complete clinical and radiography control was made on the 2nd, 4th, 6th and 8th week after the surgery. The improvement of the function of the limb, the bone axis, the consolidation of the fragments, the formation of the callus and the position of the fixator 1 was evaluated.

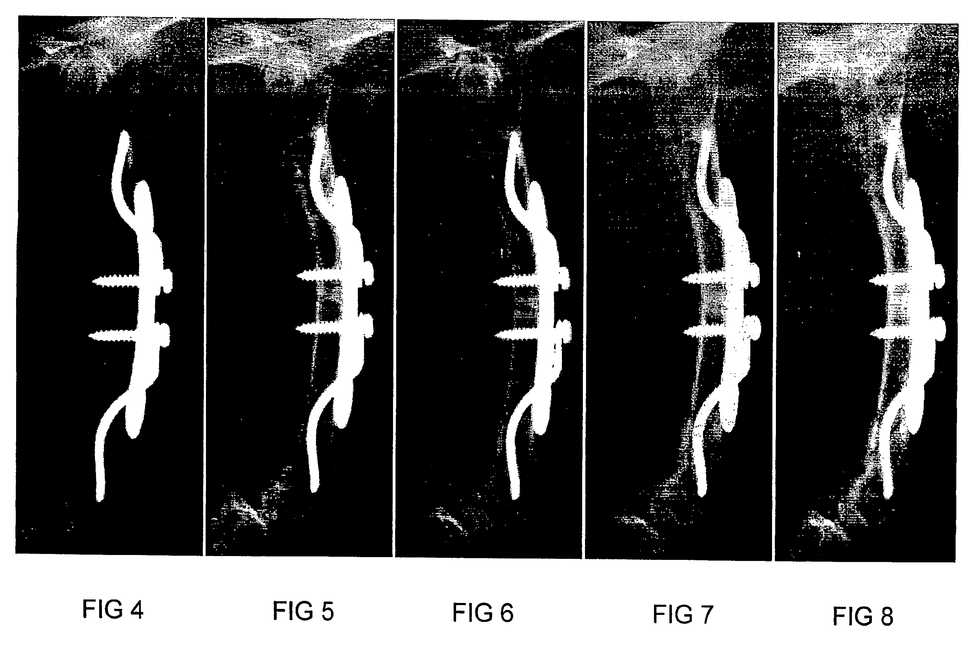

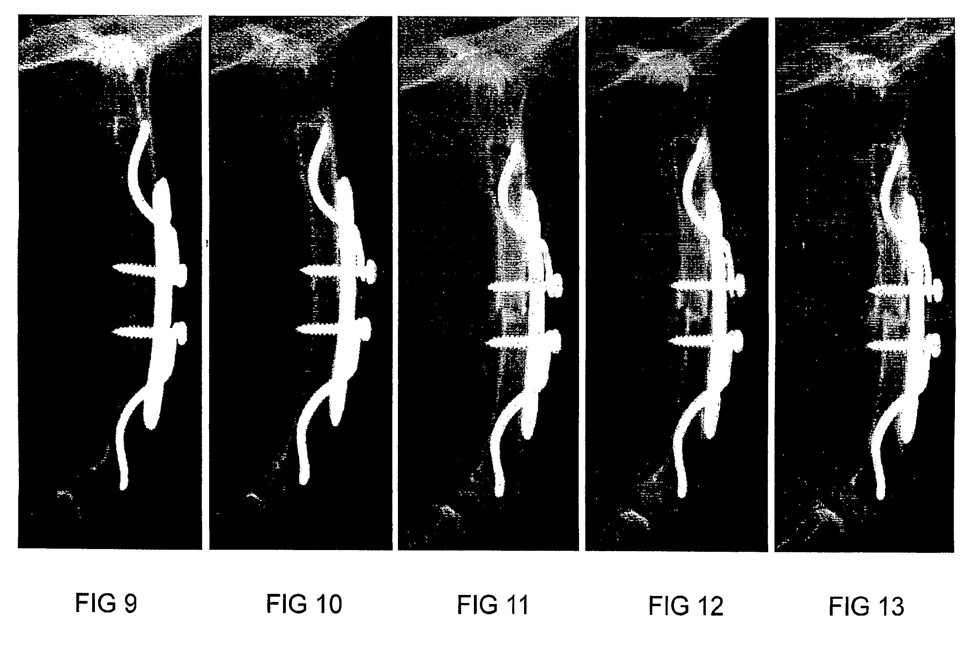

All nine described fractures were artificially modelled in the middle third of the diaphysis of the rabbits' right thighbone. The drawings fig 4 and fig 9 depict X-ray radiographs of the thighbone of two patients two weeks after the surgery. Drawings fig 5 to fig 8 and fig 10 to fig 13 are X-ray radiographs of the same patients after 4, 6 and 8 weeks. The results of the recovery were: all nine rabbits leaned on the operated leg on the next day quite strongly. The appetite and general vigour were a little lower than before the surgery. These parameters recovered in all of the test animals during the first week. On fifth day after the surgery one rabbit stopped suddenly to lean on the operated leg and started to hold it in an abnormal position. During the clinical and radiography check it appeared that there was a fragmented fracture of the distal fragment. This rabbit was excluded from the experiment.

After two weeks, no functional disorders of the limbs of the eight patients was observed during clinical evaluation.. No observable muscle atrophy occurred. The appetite and activity was normal. In radiography analysis, five rabbits featured a minimal endostial and intramedullary callus in the fracture zone. The slit between the fragments was clearly visible. No formation of periostal callus was observed. All the elements of the fixator 1 were in their position (drawing fig 5). In three rabbits, a partial shift of the bone screw was observed in the proximal fragment whereby the curved rod 3 was displaced a little and a dislocation of 1-2 mm between the fragments was formed. In the fracture zone, only endostial callus was visible. Growth of periostal callus was observed around the pointed end of the proximal bone screw 4 and near the proximal edge of the supporting plate 2. All other elements of the fixator were in their correct positions.

After four weeks, the clinical picture of all eight animals was without changes and entirely normal. In five animals, according to radiographic analysis, intense formation of endostial callus had taken place in the fracture zone and as a result the slit between the fragments had practically disappeared. In the fracture zone, a slight periostal callus was clearly visible. The position of the fixator 1 and bone screws 4 was without changes (drawing fig 6). In three rabbits, the bone screw 4 shifted even more in the proximal fragment as a result of which the tail-end 7 of the curved rod 3 lifted 2-3 mm from the supporting plate 2 and the dislocation between the fragments expanded up to 3 mm. No other mechanical changes in the position of the supporting plate 2 was observed. On the radiograph, one could clearly see an intense growth of periostal callus near the proximal edge of the supporting plate 2 and even on the supporting plate, as well as around the pointed end of the bone screw 4 and in the zone of the fracture. Endostial callus had not developed (drawing fig 11).

After six weeks, the clinical picture was without changes. The behaviour of all eight rabbits was conventional. Radiographic analysis showed that in the five rabbits the consolidation was normal. The callus was not massive and embraced all bone fragments well. No dislocation was registered. The slit between the fragments had totally disappeared. All elements of the fixator 1 were in their correct positions (drawing fig 7). In the three animals the shifting of the fixator 1 had stabilised. The bone fragments were in standstill, with a dislocation of 3 mm. The periostal callus around the fracture zone and the proximal end of the supporting plate 2 was even more massive and extended to the tail-end of the fixator 1 (drawing fig 12).

After eight weeks, all the test animals were clinically totally healthy. Radiographic analysis showed that in the five animals the fractures had healed well. The callus in the fracture zone was little but substantially dense. All elements of the fixator 1 were in their correct positions (drawing fig 8).

The radiographs of the three remaining rabbits showed that the recovery of the fracture had been slower and not reached the end phase yet. The localisation of the callus was the same but it started to change denser. The elements of the fixator 1 were in correct position. The axes of the limbs were without changes and the animals leaned freely on the operated legs (drawing fig 13).

In summary, radiographic analysis showed that the fixator 1 did not feature any impairment to the bone structures and maintained its position the same as when mounted on the bone. In three animals, the curved rod 3 partially changed its position in the intramedullary channel but maintained its fixating function. In those cases the fixator 1 was not removed nor was any additional immobilisation used. Despite that the function of the limb recovered normally. No progressive dislocation of the fragments occurred and the axis of the thighbone was not changed, merely the callus formed was more massive.

The most important factor of the fracture recovery is considered to be the maximally correct, strong and stable fixation of the ends of the broken bones.

There are several methods for fracture reduction (5). Each one of them has its positive and negative features - complicated surgery technique, the expensiveness of the fixator etc. The above-described fixator 1 has combined features and a feasibly simple construction. Taking advantage of it will expand treatment possibilities for diaphyseal bone fractures.

One advantage of the combined fixator is a relatively low cost, another is lack of special training required. The supporting plate 2 mounted on the bone is quite short, at the same time the intramedullary curved rods 3 will elongate the levers of the fixator 1 proximally and distally practically up to the metaphysis. Comparing with a plate fixation, the application of the fixator 1 will not demand such an extensive surgical access. This enables shortening of surgery time and a reduction of injury to the tissues, hence providing optimal conditions for recovery of the fracture. The curved rods 3 form a dynamical fixation in the intramedullary channel, resting on the endosteum in three points and ensure an extensive and stable contact with the bone (6). Traditional intramedullary nails will not ensure the sufficient contact with the bone, thus they provide lesser resistance to the rotation forces. This is especially true in the case of perpendicular fractures which often require additional fixators (7).

However, this is not necessary when using the present fixation. The particular feature of the above-described fixator is the original principle of fixation to the bone cortex, where a "forceps effect" is created between the supporting plate 2 and curved rods 3 when tightening the bone screws 4, leading to the cortex being pressed between the curved rods 3 and supporting plate 2. This fixing type, where the vectors of the effective forces are directed against each other, is considered in mechanics as the most solid and stable. At the same time, the fixation of the fragments is not totally rigid. As a result of the amortisation of the rods, microscopic movement occurs in the fracture zone during limb usage (8). This in turn promotes an intense callus formation and a faster consolidation of the bone (9). In the cases of application of very rigid fixators, osteoporosis has been observed, and consequent fractures after the removal of the implant (10).

When working with rabbits as test animals it is necessary to consider the peculiarities of their anatomy. The making of a tubular bone of a rabbit is very unusual as the cortical layer is relatively thin and has a very delicate structure (11). Therefore even the slightest physical overload during the surgery, or a minimal error with the choice of the diameter of the bone screw 4 may cause additional fractures of bone fragments and a failure of the experiment.

### Example 4. The design of a bridged combined fixator.

Herein we will describe an elaborated combined fixator 1, which is bridged.

The need for an elaborated fixator 1 rose from the above observation that one curved rod 3 could shift in some patients. It happened because rabbit bones were fragile and the bone screws 4 unfastened easily. The ends of bone fragments remained in ante position and the bone accreted, thanks to the construction of fixator 1. With the traditional bone plate method, a dislocation of fragment ends is frequent.

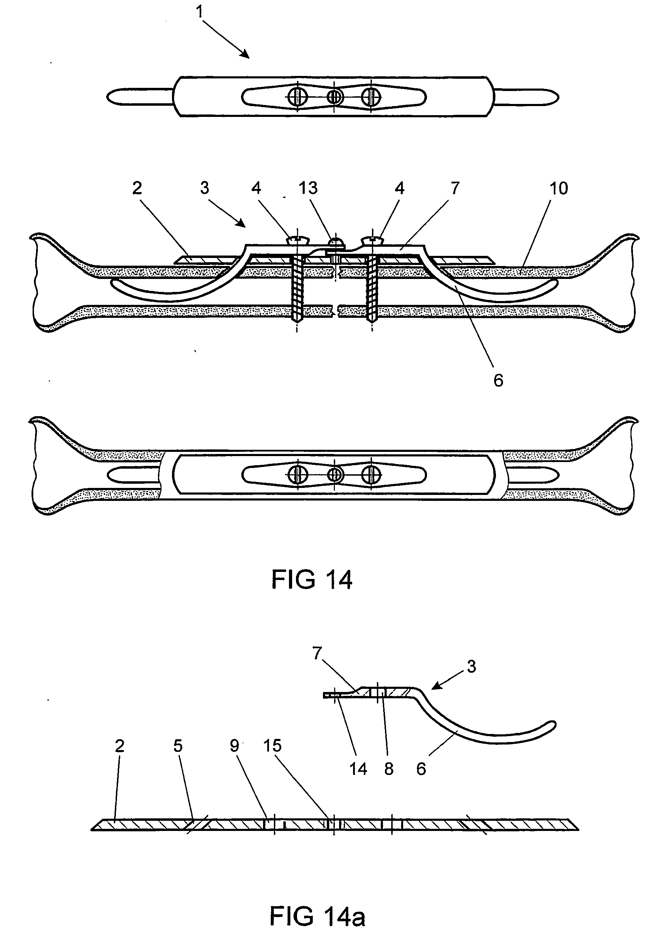

Nevertheless we wished to make the design more stable, such that the unfastening of one screw 4 would not cause the unwanted shifting of the elements. Fig 14 shows the design of a bridged fixator 1, where there is one additional element, the small screw 13. It does not get screwed into bone 10, it simultaneously joins both curved rods 3 and the supporting plate 2. There is an additional hole 14 in the curved rods 3, in the supporting plate 2 there is a threaded hole 15, which is located in the centre of the supporting plate 2.

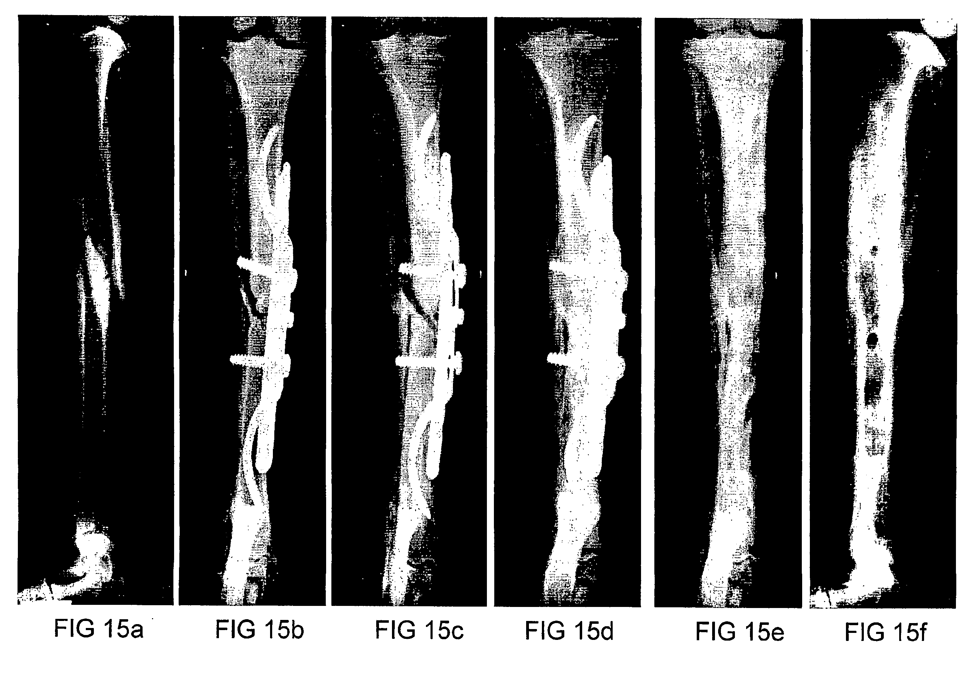

The fixing of the additional screw 13 links the curved rods 3, such as the unfastening of one screw 4 will not allow a shifting of the curved rod 3. Fig 15 depicts radiograph of a bridged fixator 1 in a dog's shinbone. The method for applying such a fixator 1 is exactly same as the surgery technique introduced in example 2. The fixing of the additional screw 13 does not take any extra measures or tools. The single difference with the bridged fixator 1 is that one always has to insert both curved rods 2. In examples 1 and 2 one could suffice with one curved rod 2 only.

### REFERENCES:

1. Seppo, A., Emits T., Mill, H.,Mõttus, J.,Seppo, T., Truupõld, U.,Yuzbashev, G. The reponator-fixator of dr.A.Seppo for osteosynthesis of fractures of long tubular bones. - 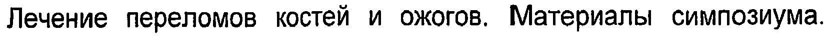 Tallinn, Valgus, p. 185, 1979.
2. Attila, M., Raekallio, M., Sandholm, M., Vaino, O. Veterinaaranestesioloogia. - Helsinki, p. 269, 1998.
3. Piermattei, D.L.,Greeley, R.G. An atlas of surgical approaches to the bones of the dog and cat. W.B.Saunders Company Philadelphia p. 162-164, 1979.
4. Brinker, W.O., Hohn, R.B., Prieur, W.D. Manual of internal fixation in small animals. - p. 33-45, 1984.
5. Brinker, W.O., Piermattei, D.L., Flo, G.L. Handbook of small animal orthopedics and fracture treatment. - W.B.Saunders Company Philadelphia, p 7-25 1991.
6. Wolff, E. F. Rush pin in veterinary orthopaedics - a review. - J Am Hosp Assoc 11: 756 - 761, 1975.
7. Vasseur, P. B., Paul, H. A., Crumley,L . Evaluation of fixation devices for prevention of rotation in transverse fractures of the canine femoral shaft : An in vitro study. - Am J Vet Res 45 : 1504-1507, 1984.
8. Uhthoff, H. K., Dubuc, F. L. Bone structure changes in the dog under rigid internal fixation. - Clin.Orthop. 81 : 165 - 170 , 1971.
9. Goodship, A. E., Kenwright, J. The influence of micromovement upon the healing of experimental tibial fractures. - J Bone Joint Surg (Br) 67-B , 650-655 , 1985.
10. Field, J. R. Bone plate fixation: its relationship with implant induced osteoporosis. - Vet. Comp. Orthop. Traum. 10 : 88-94, 1997.
11. Crigel M.,Balligand M. Critical size defect model on the femur in rabbits. - Veterinary and Comparative Orthopaedics and Traumatology 15 p 158-163, 2002.

## Claims

1. A fixator for combined metal osteosynthesis for fracture repair of long tubular bones, wherein the fixator comprises periostal connecting plate (2), having a convex cross section profile and a length of approximately 2/5 of the bone and a width of approximately 2/3 of the bone and the plate (2) having oblique planished holes (5) 5...50 mm from the ends directed extraversively and the plate (2) having holes (9) in its middle for binding screws, at least one curved rod (3) and at least one cortical screw (4), whereby
- the diameter of the curved rod (3) is chosen such to fit the curved rod (3) through the oblique hole (5) of the connecting plate (2),
- the curved part (6) of the curved rod (3) has an oval cross section profile,
- the curved part (6) of the curved rod (3) is applied intramedullary,
- the other end or tail-end (7) of the curved rod (3) is applied onto the connecting plate (2) and having a flat convex shape matching that of the plate (2),
- the tail-end (7) of the curved rod (3) contains a hole (8) for cortical screws (4), having a similar diameter to the hole (9) for the cortical screw (4) in the connecting plate (2),
- the length of the cortical screw (4) is such as to span the tail-end (7) of the curved rod (3) through the hole (9) in the plate (2) and the both cortices of the bone (10).

2. A fixator according to claim 1, **characterized in that** the shape of the curved rod (3) has a minimal conicity towards the outward ends.

3. A fixator according to claim 1 or 2, **characterized in that** comprises at least two curved rods (3) for fixing a bone fracture, one curved rod (3) applied through one hole (5) of the plate (2) and the other curved rod (3) through the other hole (5) of the plate (2).

4. A fixator according to claim 1, **characterized in that** the periostal connecting plate (2) contains in its ends additional holes for additional cortical screws to bind the plate (2) onto the bone (10).

5. A fixator according to claim 3, **characterized in that** the connecting plate (2) contains in its centre additional threaded hole (15) and the two curved rods (3) have an additional hole (14) each in the tail (7).

## Patentansprüche

1. Fixateur für eine kombinierte Metall-Osteosynthese zur Versorgung von Frakturen langer, röhrenförmiger Knochen, wobei die Fixierung aus einer periostalen Verbindungsplatte (2) besteht, die ein gewölbtes Querschnittsprofil und eine Länge von ca. 2/5 des Knochens und einer Breite von ca 2/3 des Knochens aufweist; die Platte (2) hat abgestufte, eingestanzte Löcher (5) 5...50 mm, die von den Enden aus extraversiv ausgerichtet sind; die Platte (2) hat in der Mitte Löcher (9), um Schrauben anzubringen, mindestens eine gewölbtes Stäbchen (3) und mindestens eine kortikale Schraube (4), wobei
- der Durchmesser des gewölbten Stäbchens (3) so gewählt wird, dass es durch das abgestufte Loch (5) der Verbindungsplatte (2) passt,
- der gewölbte Teil (6) des Stäbchens ein ovales Querschnittsprofil aufweist,
- der gewölbte Teil (6) des Stäbchens intramedular eingesetzt wird,
- das andere Ende (7) des gewölbten Stäbchens (3) auf die Verbindungsplatte (2) angebracht wird und eine flache, gewölbte Form hat, die an die Form der Platte (2) angepasst ist,
- das Ende (7) des gewölbten Stäbchens (3) ein Loch (9) für die kortikale Schraube (4) in der Verbindungsplatte hat,
- sich die Länge der kortikalen Schraube (4) über das Ende (7) des gewölbten Stäbchens (3) durch das Loch (9) in der Platte (2) und über die beiden Kortizes des Knochens (10) erstreckt.

2. Fixateur nach Anspruch 1, **dadurch gekennzeichnet, dass** die Form des gewölbten Stäbchens (3) läuft minimal konisch auf die äußeren Enden zu.

3. Fixateur nach Anspruch 1 oder 2, **dadurch gekennzeichnet, dass** aus mindestens zwei gewölbten Stäbchen (3) zur Fixierung einer Knochenfraktur, einem gewölbten Stäbchen (3), das in ein Loch (5) der Platte (2) angebracht wird, und aus dem anderen gewölbten Stäbchen (3), das in das andere Loch (5) der Platte (2) angebracht wird.

4. Fixateur nach Anspruch 1, **dadurch gekennzeichnet, dass** die periostale Verbindungsplatte (2) an ihren Enden zusätzliche Löcher für zusätzliche kortikale Schrauben hat, um die Platte (2) am Knochen (10) zu befestigen.

5. Fixateur nach Anspruch 3, **dadurch gekennzeichnet, dass** die Verbindungsplatte (2) in der Mitte ein zusätzliches Loch mit Gewinde (15) hat und dass die zwei gewölbten Stäbchen (3) am Ende (7) jeweils ein zusätzliches Loch (14)haben.

## Revendications

1. Fixation pour ostéosynthèse pour la réparation des fractures des os longues tubulaires, ainsi que le fixateur comprend une plaque de connexion (2) qui a un profile de section croisée et une longueur d'environ 2/5 de l'os et une ampleur d'environ 2/3 de l'os (2) et la plaque a des trous obliques (5) 5...50 mm par les extrémités dirigées de travers et la plaque (2) qui a des trous dans son centre pour les vis de fixation, au moins une tunnel incurvé (3) et au moins une vis conique (4) ainsi que
- le diamètre du tunnel incurvé (3) est choisi pour s'adapter au tunnel incurvé (3) à travers le trou oblique (5) de la plaque de connexion (2),
- la partie incurvée (6) du tunnel incurvé (3) a un profile de section croisé,
- la partie incurvée (6) du tunnel incurvé (3) est appliquée au niveau intra médullaire,
- l'autre extrémité (7) du tunnel incurvé (3) est appliquée dans la plaque de connexion (2) et a une forme convexe qui s'adapte à la plaque (2),
- l'extrémité (7) du tunnel incurvé (3) contient un trou (8) pour la vis de fixation (4) car elle a un diamètre équivalent au trou (9) pour la vis de fixation (4) dans la plaque de connexion (2),
- la longueur de la vis de fixation (4) est la même de l'extrémité (7) du tunnel incurvé (3) à travers le trou (9) dans la plaque (2) et les cortex de l'os.

2. Fixation selon la revendication 1, **caractérisé par** la forme du tunnel incurvé (3) possède une conicité minimale vers les extrémités vers l'extérieur.

3. Fixation selon la revendication 1 ou 2, **caractérisé par** il comprend au moins deux tunnels incurvés (3) pour fixer une fracture d'os, un tunnel incurvé (3) appliqué à travers un trou (5) de la plaque (2) et l'autre tunnel incurvé (3) à travers le trou (5) de la plaque (2).

4. Fixation selon la revendication 1, **caractérisé par** la plaque de connexion (2) contient dans ses extrémités les trous pour lier la plaque (2) sur l'os (10).

5. Fixation selon la revendication 3, **caractérisé par** la plaque de connexion (2) contient dans son centre un trou additionnel (15) et les deux tunnels incurvés (3) qui ont un trou additionnel (14) dans l'extrémité (7).
